# EUROPEAN PATENT APPLICATION

(11) **EP 4 455 666 A1**
(43) Date of publication of application: **30.10.2024**
(21) Application number: 22907386.1
(22) Date of filing: 09.12.2022
(51) Int. Cl.: G01N 33/543, G01N 33/48, G01N 33/53, G01N 33/569

(54) **IMMUNOCHROMATOGRAPHY TEST STRIP AND IMMUNOCHROMATOGRAPHY KIT, IMMUNOASSAY METHOD USING SAME, AND SAMPLE FILTRATION METHOD**

(30) Priority: 13.12.2021 JP 2021201692
(71) Applicant: Sekisui Medical Co., Ltd., Tokyo 103-0027 (JP)
(72) Inventor: YAJI, Shohei, Tokyo 103-0027 (JP); ITO, Shizuka, Tokyo 103-0027 (JP); OKUYAMA, Shinya, Tokyo 103-0027 (JP)
(74) Representative: Ter Meer Steinmeister & Partner
(86) International application number: PCT/JP2022/045497
(87) International publication number: WO 2023/112859

(57) **Abstract**

The present invention provides an immunochromatography test strip for detecting a detection target substance, comprising: a sample supply section to which a sample potentially containing the detection target substance is supplied; a conjugate section containing a conjugate in which a label is bound to an antibody or antigen that immunologically reacts with the detection target substance; and a detector section that captures a complex containing the detection target substance and the conjugate, wherein the detection target substance is a protein with an isoelectric point of 9.5 or higher, and either or both of the sample supply section and the conjugate section are formed on a polyester fiber pad or a polyolefin fiber pad.

## Description

### [Technical Field]

The present invention relates to an immunochromatography test strip for detecting a detection target substance composed of a protein having an isoelectric point of 9.5 or higher. The present invention also relates to an immunochromatography kit containing the immunochromatography test strip. The present invention also relates to an immunoassay method for a protein with an isoelectric point of 9.5 or higher using the immunochromatography test strip or the immunochromatography kit. The invention also relates to a sample filtration method.

Priority is claimed on Japanese Patent Application No. 2021-201692, filed December 13, 2021, the contents of which are incorporated herein by reference.

### [Background Art]

An immunoassay method is a method of measuring the level of a substance (amount, concentration, presence or absence of a substance) contained in a sample using a reaction between an antigen and an antibody. Examples of immunoassay methods include ELISA and immunochromatography.

Immunoassay methods are used to detect foreign substances or metabolites that exist in extremely small amounts in the body. Therefore, immunoassay methods need to be able to detect these foreign substances or metabolites with high sensitivity.

In Patent Literature 1, influenza viruses are detected using a sample filtration filter containing a glass material. As in the technique described in Patent Literature 1, glass materials are widely used as filters for filtration members for sample filtration. Further, many immunochromatography test strips use glass fibers as the porous material.

### [Citation List]

### [Patent Literature]

Patent Literature 1: Japanese Unexamined Patent Application Publication No. 2012-233928

### [Summary of Invention]

### [Technical Problem]

An object of the present invention is to provide an immunochromatography test strip that enables the detection of proteins with an isoelectric point of 9.5 or higher with high sensitivity, and provide an immunoassay method using the same. Another object of the present invention is to provide a method for filtering a sample in which a substance with an isoelectric point of 9.5 or higher is not adsorbed to a filter member.

### [Solution to Problem]

The present inventors found that when a protein with an isoelectric point of 9.5 or higher is allowed to contact glass fibers, the protein adsorbs to the glass fibers and the measurement sensitivity decreases. Heretofore, no discussion has been made about decrease in measurement sensitivity caused by adsorption of a detection target substance to glass fibers, and how to suppress such a decrease in measurement sensitivity.

The present inventors have conducted further studies and found that the above problem can be solved by forming either or both of the sample supply section and the conjugate section on a polyester fiber pad or on a polyolefin. Based on this finding, the present invention has been completed.

Specifically, the present invention is as follows.
<1> An immunochromatography test strip for detecting a detection target substance, comprising:
   a sample supply section to which a sample potentially containing the detection target substance is supplied;
   a conjugate section containing a conjugate in which a label is bound to an antibody or antigen that immunologically reacts with the detection target substance; and
   a detector section that captures a complex containing the detection target substance and the conjugate,
   wherein the detection target substance is a protein with an isoelectric point of 9.5 or higher, and
   either or both of the sample supply section and the conjugate section are formed on a polyester fiber pad or a polyolefin fiber pad.
<2> The immunochromatography test strip according to <1>, wherein either or both of the sample supply section and the conjugate section are formed on a polyester fiber pad.
<3> The immunochromatography test strip according to <1> or <2>, which does not include a member made of glass fiber on an upstream side of the detection unit in a sample spreading direction on the immunochromatography test strip.
<4> The immunochromatography test strip according to any one of <1> to <3>, wherein the conjugate section is formed within 13 mm from a downstream end of the polyester fiber pad or the polyolefin fiber pad in a sample spreading direction.
<5> The immunochromatography test strip according to any one of <1> to <4>, wherein the conjugate section is linearly formed on the polyester fiber pad.
<6> The immunochromatography test strip according to any one of <1> to <5>, wherein the protein with an isoelectric point of 9.5 or higher is derived from a respiratory infection virus.
<7> The immunochromatography test strip according to <6>, wherein the respiratory infection virus is a coronavirus.
<8> The immunochromatography test strip according to <7>, wherein the coronavirus is SARS-CoV-2.
<9> The immunochromatography test strip according to any one of <1> to <8>, further comprising a cationic substance.
<10> The immunochromatography test strip according to <9>, wherein the cationic substance is at least one selected from a cationic polymer, a cationic surfactant, and a metal salt.
<11> An immunochromatography kit comprising a specimen diluent containing a cationic substance and the immunochromatography test strip of any one of <1> to <10>.
<12> The immunochromatography kit according to <11>, wherein the cationic substance is at least one selected from a cationic polymer, a cationic surfactant, and a metal salt.
<13> An immunochromatography kit comprising a sample filtration filter including a member formed of polyester fiber or polyolefin fiber, and the immunochromatography test strip of any one of <1> to <10>.
<14> An immunoassay method for a protein having an isoelectric point of 9.5 or higher, comprising using the immunochromatography test strip of any one of <1> to <10> or the immunochromatography kit of any one of <11> to <13>.
<15> A filtration method for a sample containing a detection target substance, the method comprising:
   a step of filtering the sample with a filter member for sample filtration including a filter formed of polyester fiber or polyolefin fiber,
   wherein the detection target substance is a substance with an isoelectric point of 9.5 or higher.
<16> An immunoassay method for a detection target substance, comprising a step of measuring the substance with an isoelectric point of 9.5 or higher in a sample prepared by the filtration method of <15> by immunochromatography,
   wherein the substance with an isoelectric point of 9.5 or higher is a protein with an isoelectric point of 9.5 or higher.

### [Advantageous Effects of Invention]

According to the present invention, a protein with an isoelectric point of 9.5 or higher can be analyzed with high sensitivity using immunochromatography. Further, according to the present invention, it is possible to provide a sample filtration method in which can prevent a substance with an isoelectric point of 9.5 or higher from being adsorbed on a filter member.

### [Brief Description of Drawings]

FIG. 1 is a diagram illustrating a mechanism that is assumed to be ineffective in conventional techniques.
FIG. 2 is a diagram showing a presumed mechanism whereby the effect of the present invention is achieved. In this context, the structure of polyester shown in FIG. 2 is a general polyester structure shown for the purpose of explaining the principle of the present invention. The actual structure of the polyester used in the present invention is not limited to the structure of the polyester shown in FIG. 2.
FIG. 3 is a schematic diagram showing a configuration of the immunochromatography test strip produced in Production Example 1.
FIG. 4 is a schematic diagram showing an embodiment of an immunochromatography test strip including a sample pad and a conjugate pad. A linear conjugate section is disposed on the conjugate pad.
FIG. 5 is a diagram showing the line width (W) of a linear conjugate section.

### [Description of Embodiments]

In the present invention, numerical ranges expressed using "to" include the numerical values described before and after "to".

Hereinbelow, embodiments of the present invention are described, but the present invention is not limited to such embodiments, and various modifications can be made as long as such modifications do not deviate from the substance of the present invention.

### [Immunochromatography test strip]

The immunochromatography test strip according to one embodiment of the present invention is an immunochromatography test strip for detecting a detection target substance, comprising: a sample supply section to which a sample potentially containing the detection target substance is supplied; a conjugate section containing a conjugate in which a label is bound to an antibody or antigen that immunologically reacts with the detection target substance; and a detector section that captures a complex containing the detection target substance and the conjugate, wherein the detection target substance is a protein with an isoelectric point of 9.5 or higher, and either or both of the sample supply section and the conjugate section are formed on a polyester fiber pad or a polyolefin fiber pad.

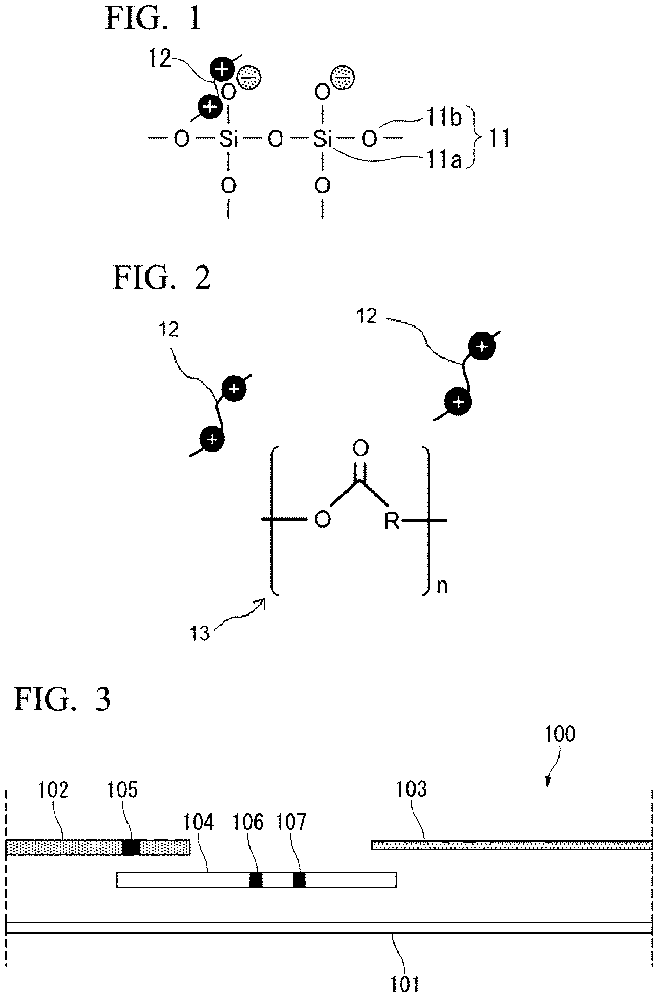

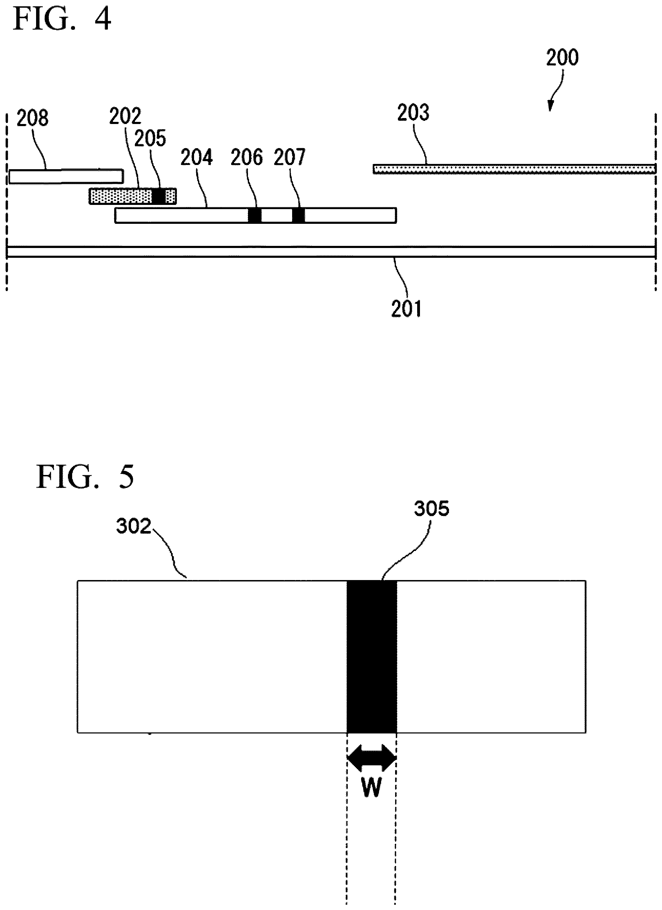

The configuration of an immunochromatography test strip according to one embodiment of the present invention is described below with reference to FIG. 3 or FIG. 4.

FIG. 3 is a schematic diagram showing the configuration of an example of the immunochromatography test strip according to the present embodiment. The immunochromatography test strip 100 shown in FIG. 3 includes a backing sheet 101, a conjugate pad 102, an absorbent pad 103, and an insoluble membrane 104. In the immunochromatography test strip 100, the conjugate pad 102, the insoluble membrane 104, and the absorbent pad 103 are arranged in this order from upstream to downstream in the flow direction of the sample. Each pad is arranged so as to at least partially overlap the pads positioned above and below. On the conjugate pad 102, disposed from upstream to downstream in the flow direction of the sample are: a sample supply section (not shown) to which a sample potentially containing the detection target substance is supplied; and a conjugate section 105 containing a conjugate in which a label is bound to an antibody or antigen that immunologically reacts with the detection target substance. Either or both of the sample supply section and the conjugate section 105 are formed on a polyester fiber pad or a polyolefin fiber pad. The conjugate pad 102 is, for example, a polyester fiber pad or a polyolefin fiber pad. A test line (detector section) 106 to capture a complex containing the detection target substance and the conjugate, and a control line 107 are provided on the insoluble membrane 104.

FIG. 4 is a schematic diagram showing the configuration of another example of the immunochromatography test strip according to the present embodiment. The immunochromatography test strip 200 shown in FIG. 4 includes a backing sheet 201, a conjugate pad 202, an absorbent pad 203, an insoluble membrane 204, and a sample pad 208. In the immunochromatography test strip 200, the sample pad 208, the conjugate pad 202, the insoluble membrane 204, and the absorbent pad 204 are arranged in this order from upstream to downstream in the flow direction of the sample. Each pad is arranged so as to at least partially overlap the pads positioned above and below. On the sample pad 208 is provided a sample supply section (not shown) to which a sample potentially containing the detection target substance is supplied. On the conjugate pad 202 is provided a conjugate section 205 containing a conjugate in which a label is bound to an antibody or antigen that immunologically reacts with the detection target substance. Either or both of the sample supply section and the conjugate section 205 are formed on a polyester fiber pad or a polyolefin fiber pad. Either or both of the sample pad 208 and the conjugate pad 202 are, for example, a polyester fiber pad or a polyolefin fiber pad. A test line (detector section) 206 to capture a complex containing the detection target substance and the conjugate, and a control line 207 are provided on the insoluble membrane 204.

In the immunochromatography test strip 100 (200) of the present embodiment, it is preferable that no glass fiber member be included on the immunochromatography test strip 100 (200) at a position upstream of the test line (detector section) 106 (206) in the sample spreading direction.

### (Protein with an isoelectric point of 9.5 or higher)

The detection target substance in the present invention is a protein with an isoelectric point of 9.5 or higher. The protein with an isoelectric point of 9.5 or higher is not particularly limited as long as it can be detected using an antigen-antibody reaction, and examples thereof include exogenous substances (viruses, bacteria, administered drugs, etc.), proteins derived from exogenous substances (metabolites of exogenous substances, secretions by bacteria, etc.), and endogenous substances (antibodies, hormones, etc. produced within the body). The protein with an isoelectric point of 9.5 or higher also encompasses protein fragments, i.e., peptide fragments with an isoelectric point of 9.5 or higher.

The detection target substance may be a protein with an isoelectric point of 9.5 or higher, 9.6 or higher, 9.7 or higher, 9.8 or higher, 9.9 or higher, or 10.0 or higher. The isoelectric point of a protein can be calculated by inputting UniProtKB into Expasy Compute pI/Mw (https://www.expasy.org/resources /compute-pi-mw) or by isoelectric focusing.

Hereinbelow, a protein with an isoelectric point of 9.5 or higher may be simply referred to as a detection target substance.

The detection target substance in the present invention is preferably a virus, more preferably a respiratory infection virus, even more preferably a coronavirus, most preferably SARS-CoV-2. SARS-CoV-2 is a virus that causes the coronavirus disease 2019 (COVID-19).

### (Sample)

In the immunoassay method of the present embodiment, examples of samples that potentially contain the detection target substance mainly include a biological sample derived from an organism, an extract obtained by extracting the detection target substance from a biological sample, and the like. Further examples of usable samples that potentially contain the detection target substance include food samples such as liquid beverages, semi-solid foods, and solid foods, samples from natural environments such as soil, rivers, and seawater, and wipe samples from factory production lines or clean rooms.

In the immunoassay method of the present embodiment, a biological sample is preferable as the sample that potentially contain the detection target substance. More specific examples of biological samples include blood, serum, plasma, urine, tears, ear discharge, prostatic fluid, and respiratory secretions. Respiratory secretions are more preferred. In the context of the present specification, the term "respiratory secretion" means bodily fluids secreted in or on the tissues of the respiratory organs. Examples of respiratory secretions include bodily fluids secreted in the nostrils, nasal cavities, pharynx, nasopharynx, and oral cavity, and particularly preferred examples include nasopharyngeal swabs, nasal swabs, saliva, and sputum. In the context of the present specification, "respiratory organ" is a generic term for organs related to respiration, and covers organs ranging from the nasal vestibule to the alveoli (lungs) via the nasal cavity, pharynx, larynx, trachea, bronchi, and bronchioles.

Examples of subjects from which the biological sample is to be collected include humans or animals (e.g., monkeys, dogs, and cats), of which humans are preferable. The biological sample may be a biological sample as it is taken from a subject, or may be a sample obtained by subjecting a collected biological sample to treatments such as dilution and concentration that are usually performed. The person who collects and prepares a biological sample used in the present invention may or may not be identical to the person who performs the immunoassay method of the present embodiment. Further, the biological sample used in the immunoassay method of the present embodiment may be one collected or prepared during implementation of the immunoassay method of the present embodiment, or one previously collected or prepared and stored.

When the biological sample contains SARS-CoV-2 or its peptide fragments as a detection target substance, its concentration is 0.1 to 100,000 TCID50/mL, 1 to 50,000 TCID50/mL, or 10 to 10,000 TCID50/mL.

### (Label)

In the present specification, the term "label" means a substance that generates a signal in the detector section when directly or indirectly bound to a detection target substance. The signal derived from the label may be measured according to a known method. The signal derived from the label can be measured, for example, by measuring the absorbance or the intensity of reflected light. The signal derived from the label may be examined visually or using a specific measuring device.

As the label used in the immunochromatography test strip of the present embodiment, known labels that have been conventionally used in immunochromatography test strips can be used. As the label, colloidal metal particles such as colloidal gold particles or colloidal platinum particles, colored latex particles, or magnetic particles are preferable, and colloidal gold particles are particularly preferable. In order to be detected in the detector section, the label is preferably modified with a tag or the like, or has an antibody or antigen immobilized thereon that immunologically reacts with the detection target substance.

It is preferable to use a label with an appropriate particle size depending on the type of label. For example, when colloidal gold particles are used as a label, the particle size is preferably 20 to 70 nm, particularly preferably 45 to 65 nm. The above colloidal gold particles can be produced by a generally known method, for example, by dropping an aqueous trisodium citrate solution into a heated aqueous tetrachloroauric(III) acid solution and stirring.

### (Antibody or antigen that immunologically reacts with detection target substance)

The immunochromatography test strip of the present embodiment uses a label on which an antibody or antigen that immunologically reacts with the detection target substance is immobilized. Hereinbelow, a label on which an antibody or antigen that immunologically reacts with the detection target substance is immobilized may also be referred to as "conjugate". In the present specification, immobilizing an antigen or antibody means physically or chemically supporting the antigen or antibody on a label or an insoluble membrane.

When an antibody is immobilized on a label, it is more preferable that an antibody that immunologically reacts with the detection target substance immobilized on the label and an antibody that immunologically reacts with the detection target substance immobilized on the detector section are different. In this context, "different" means that types are different, and different antibodies recognize different epitopes. When an immunochromatography test strip is prepared using different antibodies, i.e., one immobilized on the label and the other on the detector section, it becomes possible to suppress competition between the reaction between the detection target substance and the antibody in the detector section and the reaction with unreacted conjugate. Further, it also becomes possible to increase the reactivity between the detection target substance bound to the conjugate and the antibody in the detector section. As a result, the sensitivity of the immunochromatography test strip improves.

The antibody used in the immunoassay method of the present embodiment may be either a monoclonal antibody or a polyclonal antibody, both of which can be produced according to known methods. The antibody used in the immunoassay method of the present embodiment is preferably a monoclonal antibody. The use of a monoclonal antibody can increase the specificity of the reaction.

Further, in addition to the entire molecules of these antibodies, functional fragments of antibodies having antigen-antibody reaction activity are also included in the "antibodies" in the context of the present specification. Examples of functional antibody fragments include those obtained through the process of immunizing animals, those obtained using genetic recombination techniques, and chimeric antibodies. Specific examples of functional antibody fragments include F(ab')₂, Fab', and the like. These functional fragments can be produced by treating the antibody with a proteolytic enzyme (e.g., pepsin or papain).

### (Conjugate)

The label, i.e., the conjugate, on which an antibody or antigen that immunologically reacts with the detection target substance is immobilized, used in the immunochromatography test strip of the present embodiment, is preferably one in which a monoclonal antibody that binds to a protein with an isoelectric point of 9.5 or higher is immobilized on colloidal gold particles.

Examples of methods for immobilizing antibodies or antigens that immunologically react with the detection target substance on a label include physical adsorption, chemical bonding, and the like. General practice is to immobilize by physical adsorption. For example, for immobilizing a monoclonal antibody that binds to a protein with an isoelectric point of 9.5 or higher to colloidal gold particles, the colloidal gold particles and the monoclonal antibody that binds to a protein with an isoelectric point of 9.5 or higher are usually added to a buffer solution, to thereby implement immobilization by physical adsorption. In this process, the antibody concentration is preferably adjusted to 20 to 100 µg/mL.

The conjugate section in which the conjugate is placed may be provided linearly on the sample pad, or may be provided as a conjugate pad (described below) between the sample pad and the insoluble membrane.

### (Sample pad)

In the immunochromatography test strip of the present embodiment, a pad-shaped porous material capable of holding a conjugate can be used as a sample pad. The sample pad is formed of polyester fibers or polyolefin fibers. The sample pad has a sample supply section in a part thereof. The sample supply section is a part that supplies a sample potentially containing the detection target substance, and the side where the sample supply section exists is the upstream side of the sample pad. The sample supplied to the sample supply section is spread on the sample pad and reaches the label section.

The sample pad may include both of the sample supply section and a conjugate section described below. In this instance, a pad comprising both of the sample supply section and the conjugate section serves simultaneously as the sample pad and the conjugate pad.

### (Conjugate section)

In the present specification, the term "conjugate section" means a section of the immunochromatography test strip where the conjugate is placed. The conjugate section is formed on polyester fibers or polyolefin fibers.

The conjugate section may be present in the form of a line on the sample pad, or may be present as a conjugate pad separate from the sample pad between the sample pad and the insoluble membrane. In the conjugate pad, the conjugate may be disposed entirely or in linear form. The conjugate section can also be present on an insoluble membrane. The conjugate section is preferably present in linear form on the sample pad or the conjugate pad.

The linear conjugate section is preferably disposed in a line in a direction perpendicular to the sample spreading direction, that is, a line connecting the center of the sample supply section of the sample pad and the center of the downstream end of an insoluble membrane described below. In other words, the linear conjugate section is preferably disposed in a line in a direction perpendicular to the longitudinal direction of the sample pad and the insoluble membrane.

FIG. 5 shows the conjugate pad 302 and the linear conjugate section 305 provided on the conjugate pad 302. The line width W of the linear conjugate section 305 is not limited as long as it is wide enough to contain the conjugate in an amount necessary for detection of the detection target substance, and is preferably 2 to 8 mm, more preferably 3 to 7 mm. In the present specification, the length of the linear conjugate section in the sample spreading direction is referred to as "line width". The conjugate section is preferably formed such that the center of the conjugate section in the sample spreading direction is located within 13 mm from the downstream end of the polyester fiber pad or polyolefin fiber pad forming the conjugate pad in the sample spreading direction.

If necessary, the conjugate pad may contain "control reagents" to ensure the reliability of immunochromatography, such as antibodies that do not react with sample components labeled with a label, or highly antigenic proteins such as KLH (limpet hemocyanin) labeled with a label. These control reagents are components (substances) that are unlikely to exist in the sample, and can be selected as appropriate.

### (Insoluble membrane)

The immunochromatography test strip of the present embodiment includes at least one detection section that captures a complex containing the detection target substance and the conjugate. Preferably, the insoluble membrane includes the detection section. It is preferable that the detector section has a linear shape. It is preferable that the detector section has immobilized thereon an antibody or an antigen, which immunologically reacts with a detection target substance. When the detector section has a linear shape, the detector section is preferably disposed in a line in a direction perpendicular to the sample spreading direction, that is, a line connecting the center of the sample supply section of the sample pad and the center of the downstream end of the insoluble membrane. In other words, the linear detector section is preferably disposed in a line in a direction perpendicular to the longitudinal direction of the insoluble membrane. Immobilization of an antibody that immunologically reacts with a detection target substance to the insoluble membrane can be carried out by a conventionally known method.

Further, in consideration of measurement sensitivity, it is preferable that the immunochromatography test strip of the present embodiment does not include a member formed of glass fibers at a position upstream of the detection section.

For example, immobilization of an antibody or antigen onto an insoluble membrane can be performed as follows. A solution containing the above antibody or antigen at a predetermined concentration is prepared. Next, using a device having a mechanism capable of discharging a liquid from a nozzle at a constant speed, the liquid is linearly applied to the insoluble membrane, for example, with a line width of 0.5 to 5 mm or 0.5 to 2 mm. By drying the applied solution, an insoluble membrane on which an antibody or antigen is immobilized can be obtained. In this specification, the length of the linear detector section in the sample spreading direction is referred to as "line width". The line width of the detector section preferably shows approximately the same width over the entire line (that is, the width is ±0.2 mm over the entire line).

Part of the linear detector section may protrude toward the downstream and/or upstream side. In such a case, either the non-protruding portion or the protruding portion may have the above line width, but it is preferable that the non-protruding portion has the above line width.

Further, it is not necessary to include antibodies or antigens that immunologically react with the detection target substance over the entire linear detector section, and there may be portions that do not contain such immunologically reactive antibodies or antigens as long as the effect of this embodiment is obtained. Examples of potential embodiments with portions where the line does not contain antibodies or antigens that immunologically react with the detection target substance include a case where the line is formed of dots, and a case where a plurality of thin lines (for example, with line width 1 mm) are formed.

Further, the solution containing the antibody or antigen at a predetermined concentration can be prepared by adding the antibody or antigen to a buffer solution. Examples of buffer solution include commonly used buffers such as phosphate buffer, Tris buffer, and Good's buffer. The pH of the buffer solution is preferably in the range of 6.0 to 9.5, more preferably 6.5 to 8.5, even more preferably 7.0 to 8.0. The buffer solution may further contain salts such as sodium chloride, stabilizers or preservatives such as sucrose, preservatives such as proclin, and the like.

On the insoluble membrane, a control capture reagent conventionally used in immunochromatography test strips may be immobilized. The control capture reagent is a reagent for ensuring the reliability of the assay, and captures the control reagent contained in the conjugate or the conjugate section.

Examples of membranes forming the insoluble membrane used in the present invention include pads formed of nonwoven fibers such as paper, cellulose blends, nitrocellulose, polyester, acrylonitrile copolymers, rayon, and the like. Preferably, membranes formed of glass fibers are not used.

The total length of the insoluble membrane, that is, the length from the upstream end to downstream end of the insoluble membrane, can be adjusted to an appropriate length by those skilled in the art. Specifically, the length may be adjusted to 15 to 40 mm, 18 to 35 mm, or 20 to 30 mm.

### (Absorbent pad)

In the immunochromatography test strip of the present embodiment, an absorbent pad is preferably provided at the downstream end of the insoluble membrane. The absorbent pad is a part having liquid absorbency that controls the development of the sample by absorbing the sample that has been transferred and passed through the insoluble membrane. As the absorbent pad, a known absorbent pad conventionally used in immunochromatography test strips can be used, and for example, filter paper can be used.

The total length of the absorbent pad, that is, the length from the upstream end to downstream end of the absorbent pad, can be adjusted to an appropriate length by those skilled in the art. Specifically, the length may be adjusted to 5 to 100 mm, 20 to 80 mm, or 20 to 60 mm.

### (Immunochromatography test strip)

The immunochromatography test strip is preferably prepared by disposing a sample pad, an insoluble membrane, etc. on a solid support such as a plastic adhesive sheet. The solid support is composed of a material that does not interfere with the capillary flow of the sample and the conjugate. Alternatively, the immunochromatography test strip may be immobilized on the solid support using an adhesive or the like. In this instance, the adhesive components are also composed of substances that do not interfere with the capillary flow of the sample and conjugate. In this context, it is also possible to laminate a polyester film or the like as a top film for the purpose of increasing the mechanical strength of the insoluble membrane and preventing water evaporation (drying) during the assay. The immunochromatography test strip may be stored or mounted in an appropriate container (housing) that accommodates requirements in terms of the size of the test strip, the method and position of adding the sample, the position where the detector section of the insoluble membrane is formed, the method of detecting the signal, etc., and the test strip in a form stored or mounted in this manner is called a "device".

The immunochromatography test strip of the present embodiment includes a sample pad and an insoluble membrane, and may further include other reagents and members depending on the measurement conditions and sample. Examples of other reagents include blocking agents to prevent non-specific reactions, and examples of other members include additional pads to remove components unnecessary for measurement in the sample. For example, an additional pad can be provided between the sample pad and the porous membrane to allow the detection target substance or the complex containing the detection target substance to spread smoothly over the insoluble membrane.

### (Polyester)

In the present specification, the term "polyester" means a polycondensate synthesized through dehydration-condensation of a polyhydric carboxylic acid and a polyhydric alcohol whereby an ester bond is formed. The term "polyester fiber" means a fiber prepared from polyester.

As the polyester fiber, polyester fibers prepared from polyethylene terephthalate, polytrimethylene terephthalate, polybutylene terephthalate, or polyethylene naphthalate can be used.

### (Polyolefin)

In the present specification, the term "polyolefin" means a compound in which structural units derived from alkene are repeatedly connected. The term "polyolefin fiber" means a fiber prepared from polyolefin.

As the polyolefin fiber, for example, polyolefin fibers prepared from polypropylene, polyethylene, or polystyrene can be used.

The polyester fiber or polyolefin fiber may be treated with an organic binder such as an acrylic resin.

Polyester fibers and polyolefin fibers do not have polarity within their structures. Therefore, it is presumed that the adsorption of substances having an isoelectric point of 9.5 or higher is reduced compared to polar materials such as glass fibers, which are described below. Since the above effect was obtained with polyester fibers as well, it is presumed that the same effect can be obtained when polyolefin fibers are used.

The polyester fiber pad or polyolefin fiber pad may contain constituent fibers other than polyester fibers or polyolefin fibers as long as the effects of the present invention are obtained, but it is preferable to use a pad formed only of polyester fibers or a pad formed only of polyolefin fibers. Examples of constituent fibers other than polyester fibers or polyolefin fibers include synthetic fibers such as polyamide or acrylic polymers. Examples of natural fibers include cotton and linen.

### (Glass fiber)

In the present specification, the term "glass fiber" means a fiber using silicon dioxide (SiO₂) as a constituent component. In the glass 11 having silicon dioxide (SiO₂) as a constituent component, a network structure based on SiO4 tetrahedrons with silicon atoms 11a and oxygen atoms 11b positioned at vertices is formed at a side of temperature lower than the glass transition point (FIG. 1). In this network structure, the oxygen atoms 11b bonded to the silicon atoms 11a are negatively charged. It is assumed that the protein 12 having an isoelectric point of 9.5 or higher is adsorbed to this negatively charged oxygen atom 11b, resulting in a decrease in measurement sensitivity (FIG. 1).

In contrast, in the present invention, by forming either one or both of the sample supply section and the conjugate section on a fiber pad formed of polyester or polyolefin 13, adsorption of the protein 12 with an isoelectric point of 9.5 or higher to the sample supply section or the conjugate section can be suppressed, which presumably prevents the decrease in the measurement sensitivity (FIG. 2).

### (Cationic substance)

In the present specification, the term "cationic substance" means a substance that has a cationic functional group and becomes positively charged when it comes into contact with water. When the cationic substance has both a cationic functional group and an anionic functional group, it is sufficient that the entire molecule is positively charged. The cationic substance is preferably present when the detection target substance is being bound to an antibody or antigen that immunologically reacts with the detection target substance.

Examples of cationic functional groups include amino groups such as a primary amino group, a secondary amino group, and a tertiary amino group; onium base groups such as a quaternary ammonium group, and a phosphonium group; amino acid residues such as an arginyl group, a lysyl group, a histidyl group, and a guanidyl group; and heterocyclic groups such as an imidazole group.

The cationic substance may be, for example, at least one selected from the group consisting of metal salts, cationic polymers, cationic surfactants, and cationic peptides. The cationic substance preferably is a metal salt, a cationic polymer, or a cationic surfactant, more preferably a cationic polymer or a cationic surfactant, even more preferably a cationic polymer.

### (Metal salt)

As the metal salt, any metal salt that generates metal ions when ionized in an aqueous solution can be used without any limitation. Even without a cationic functional group as described above, a metal salt that ionizes when it comes into contact with water becomes a cationic metal ion. Examples of metal ions include alkali metal ions, alkaline earth metal ions, and other metal ions, with alkali metal ions and alkaline earth metal ions being preferred. Examples of alkali metal ions include lithium ions, sodium ions, potassium ions, and the like. Examples of alkaline earth metal ions include magnesium ions, calcium ions, and the like. Examples of other metal ions include copper ions, and the like. Sodium ions are preferred as alkali metal ions, and magnesium ions are preferred as alkaline earth metal ions. The alkali metal salt is preferably sodium chloride or sodium citrate, and the alkaline earth metal salt is most preferably magnesium sulfate.

### (Cationic polymer)

In the present specification, the term "cationic polymer" means a polymer having a cationic functional group as a side chain. The cationic functional group as the side chain is preferably a quaternary ammonium group. The cationic polymer is preferably a cationic polymer having hexadimethrine bromide (CAS RN28728-55-4) or 2-methacryloyloxyethyl phosphorylcholine as constituent units, most preferably a cationic polymer having 2-methacryloyloxyethyl phosphorylcholine as constituent units, which exhibits a kinematic viscosity of 20 to 60 m²/s at 25 °C in the form of an aqueous solution with a concentration of 0.5 % by mass, exhibits a surface tension of 70 × 10⁻³ to 80 × 10⁻³ N/m at 25 °C in the form of an aqueous solution with a concentration of 0.1 % by mass, while having a mass average molecular weight (Mw) of 50 to 600 kDa. In this context, the cationic polymer having 2-methacryloyloxyethyl phosphorylcholine as constituent units is commercially available as Lipidure-BL502 (registered trademark).

The kinematic viscosity η [m²/s] of the aqueous solution of the cationic polymer at a concentration of 0.5 % by mass at 25°C can be obtained by dividing the viscosity µ [Pa•s] by the density ρ [kg/m³], wherein the viscosity µ is determined by measuring an aqueous solution in which the cationic polymer is dissolved in water at a concentration of 0.5 % by mass with an Ubbelohde viscometer at 25 °C, and the density ρ is a density of the same aqueous solution at 25 °C.

The surface tension γ [mN/m] of a 0.1 % by mass aqueous solution of the cationic polymer at 25 °C can be determined by measurement at 25 °C by du Noüy ring method, Wilhelmy plate method, or drop weight method with respect to an aqueous solution in which the cationic polymer is dissolved in water at a concentration of 0.1 % by mass.

The mass average molecular weight (Mw) of the cationic polymer can be determined by gel permeation chromatography (GPC). Polyethylene glycol is used as a standard substance.

### (Cationic surfactant)

In the present specification, the term "cationic surfactant" means a surfactant whose portion to which a hydrophobic group is bonded is positively charged when dissolved in an aqueous solution. Examples of cationic surfactants include alkylamine salt type surfactants and quaternary ammonium salt type surfactants.

Examples of alkylamine salt type surfactants include salts of amines having 1 to 3 alkyl groups each having 8 to 22 carbon atoms, such as monododecylamine, monooctadecylamine, dioctadecylamine, and triotadecylamine, with inorganic acids such as hydrochloric acid and sulfuric acid, or lower carboxylic acids such as acetic acid, lactic acid, and citric acid. Specific examples thereof include stearylamine acetate [CAS number: 2190-04-7, product name: Acetamin 86, manufactured by Kao Corporation].

Examples of quaternary ammonium salt type surfactants include salts of ammonium having 1 to 3 alkyl groups each having 8 to 22 carbon atoms, such as dodecyltrimethylammonium, octadecyltrimethylammonium, hexadecyltrimethylammonium, didecyldimethylammonium, and benzylmethyltetradecylammonium, with chlorine, bromine, etc. Specific examples thereof include lauryltrimethylammonium chloride [CAS number: 112-00-5, product name: Quartamin 24P, manufactured by Kao Corporation], and dodecyltrimethylammonium bromide [CAS number: 1119-94-4, manufactured by Tokyo Chemical Industry Co., Ltd.].

### (Cationic peptide)

The term "cationic peptide" means a peptide that exhibits cationicity when dissolved or dispersed in water. The cationic peptide may contain many basic amino acid residues (arginine (Arg), lysine (Lys), or histidine (His)) among the amino acid residues constituting the peptide.

The concentration of the cationic substance can be adjusted as appropriate depending on the type of target substance to be detected, the type of sample, and the type of immunoassay method. Examples of the concentration are given below. The following concentrations are applicable to both of the case where the cationic substance is included in the specimen diluent and the case where the cationic substance is added to the immunochromatography test strip instead of the specimen diluent.

In the case of a metal salt, the concentration of the cationic substance is, for example, 0.1 to 1,000 mM, 1 to 750 mM, 2 to 500 mM, or 50 to 500 mM, based on the specimen diluent or the solution added to the immunochromatography test strip. Further, in the case of a cationic polymer, a cationic surfactant, or a cationic peptide, the concentration is, for example, 0.0001 to 20 % by mass, 0.0005 to 10 % by mass, 0.001 to 5 % by mass, 0.01 to 1 % by mass, or 0.01 to 0.5 % by mass, based on the specimen diluent or the solution added to the immunochromatography test strip.

### [Immunochromatography kit]

The immunochromatography kit of the present embodiment includes a specimen diluent and the immunochromatography test strip described above.

### (Specimen diluent)

Although it is preferable that the specimen diluent contains a cationic substance at the time of sale, the immunochromatography kit may contain a cationic substance separately from the specimen diluent. In such case, a person performing immunochromatography can mix the cationic substance and the specimen diluent. In this context, the cationic substance being "contained in the specimen diluent" should be regarded as encompassing an embodiment in which the cationic substance is dissolved in the specimen diluent at the time of sale, and an embodiment in which the cationic substance is not dissolved in the sample dilution liquid at the time of sale, but is dissolved in the specimen diluent by a person performing immunochromatography at the time of measurement.

Preferably, the specimen diluent contains a buffer. The term "buffer" means a solution having a pH-buffering effect.

Non-limiting examples of buffers that can be used as appropriate in the present invention include known buffers such as PBS (phosphate buffered saline), MES (2-(N-morpholino)ethanesulfonic acid), PIPES (piperazine-N,N'- bis(2-ethanesulfonic acid), ACES (N-(2-acetamido)-2-aminoethanesulfonic acid), ADA (N-(2-acetamido)iminodiacetic acid), Bis-Tris(2,2-bis(hydroxyethyl)-(iminotris-(hydroxymethyl)-methane), Tris (tris(hydroxymethyl)aminomethane), MOPS (3-morpholinopropanesulfonic acid), HEPES (4-(2-hydroxyethyl)-1-piperazineethanesulfonic acid), a citric acid buffer, a glycine buffers, a borate buffer, and a phosphate buffer. The buffer used in the present invention is preferably a phosphate buffer.

The immunochromatography kit of the present embodiment may also include other test reagents and/or an instruction manual, etc., such as a specimen filtration filter, a cotton swab for sample collection, a standard antigen substance, and a quality control antigen sample.

### [Sample filtration method]

The sample filtration method of the present embodiment is a method for filtering a sample containing a detection target substance, and includes a step of filtering the sample with a filter member for sample filtration including a filter formed of polyester fiber or polyolefin fiber. The detection target substance is a substance with an isoelectric point of 9.5 or higher.

The sample filtration method of the present embodiment can prevent a detection target substance from being adsorbed to a specimen filtration filter. Therefore, the filtration method of the present invention can prevent a detection target substance from being adsorbed to a specimen filtration filter before the measurement or detection in a wide range of applications such as immunoassays and nucleic acid detection.

The sample filtration method of the present embodiment includes the step of filtering the sample with a filtration member for sample filtration including a filter formed of polyester or polyolefin. The filtration member may include one filter formed of polyester fiber or polyolefin fiber, or may include two or more such filters. The retention particle size, thickness, etc. of the filter are not particularly limited as long as the effects of the present invention can be obtained.

As described in Patent Literature 1, there are many filtration members that employ glass fibers as the filter material of the filtration member for sample filtration. The present invention filters a sample with a filter member for sample filtration including a filter formed of polyester fiber or polyolefin fiber, and can thereby prevent substances having an isoelectric point of 9.5 or higher (proteins, nucleic acids, etc.) from being adsorbed to the glass fibers.

The sample filtration method of the present embodiment may include a step of diluting the sample with a sample diluent, if necessary, before the filtration step.

When the substance with an isoelectric point of 9.5 or higher is a protein with an isoelectric point of 9.5 or higher, substances with an isoelectric point of 9.5 or higher in a sample prepared by filtering the sample through a filter member for specimen filtration including a filter made of polyester or polyolefin can be measured by immunochromatography.

### EXAMPLES

Hereinbelow, the present invention is described in detail with reference to examples, which however should not be construed as limiting the present invention. In the following description, the unit "%" refers to "% by mass", unless otherwise specified.

### <<Production Example 1>> Preparation of immunochromatography test strip for SARS-CoV-2 measurement

### 1) Preparation of anti-SARS-CoV-2 monoclonal antibody

Anti-SARS-CoV-2 monoclonal antibodies used in the following tests were obtained by immunizing mice with recombinant SARS-CoV-2 nuclear proteins and using a method commonly used by those skilled in the art to produce monoclonal antibodies.

### 2) Preparation of colloidal gold-labeled anti-SARS-CoV-2 antibody (conjugate)

1 mL of phosphate buffer containing 25 µg/mL of anti-SARS-CoV-2 antibody was added to 20 mL of 1 OD/mL-colloidal gold solution, and the resulting mixture was stirred at room temperature for 10 minutes. Subsequently, 2 mL of 10 % BSA solution was added to the colloidal gold solution and the resulting was stirred at room temperature for 5 minutes. The obtained solution was centrifuged at 10,000 rpm for 45 minutes at 10 °C, and the supernatant was removed. The residue was suspended in Conjugate Dilution Buffer (Scripps) to obtain a gold colloid-labeled anti-SARS-CoV-2 antibody solution.

### 3) Preparation of conjugate pad

The colloidal gold-labeled antibody solution prepared in 2) was diluted to 4 OD/mL with a 1.33 % casein, 4 % sucrose solution (pH 7.5) to prepare a conjugate solution. The conjugate solution was applied linearly with a line width of 5 mm onto a glass fiber pad and dried to obtain a conjugate pad.

### 4) Preparation of antibody-immobilized membrane

PBS containing 0.75 mg/mL anti-SARS-CoV-2 antibody and 2.5 % sucrose was prepared and used as a test line coating solution. PBS containing 1.0 mg/mL goat anti-mouse IgG monoclonal antibody and 2.5 % sucrose was prepared and used as a control line coating solution. Using a dispenser for immunochromatography "XYZ3050" (manufactured by BioDot), the test line coating solution and the control line coating solution were each applied on a nitrocellulose membrane at 1.0 µL/cm and dried to obtain an antibody-immobilized membrane.

### 5) Production of immunochromatography test strip

An immunochromatography test strip for measuring SARS-CoV-2 (hereinafter simply referred to as "immunochromatography test strip" as well) was prepared by attaching the antibody-immobilized membrane, the conjugate pad, and the absorption pad on a plastic adhesive sheet and cutting the resulting into a 5 mm-width strip.

The configuration of this immunochromatography test strip is shown in FIG. 3. In the immunochromatography test strip 100 of this production example, the backing sheet 101 is formed of a plastic adhesive sheet.

### «Example 1» Evaluation 1 of the influence of conjugate pad material on virus antigen measurement sensitivity

Samples were prepared by adding the following antigens to the sample diluent provided with RapidTesta FLU/NEXT (Sekisui Medical Co., Ltd.).
Influenza A virus (FLU A) antigen: derived from Kitakyusyu 159/93 strain
Influenza B virus (FLU B) antigen: derived from Lee 40 strain
RS virus antigen: RSV Antigen, Long strain inactivated antigen (Meridian)
Adenovirus antigen: Adenovirus Antigen (Fitzgerald)
SARS-CoV-2 antigen: SARS-CoV-2 Nucleocapsid Recombinant Protein (Icosagen)

In the sample, a conjugate pad material of 140 mm² per 120 µL of the sample was immersed for 10 minutes to obtain a pad member immersion sample. The sample and the pad member immersion sample were measured under the conditions shown in Table 1, and the absorbance of the test line was calculated using a RapidTesta Reader (Sekisui Medical Co., Ltd.). The antigen recovery rate when the pad member was immersed in the sample was calculated by the following formula. Recovery rate (%) = Absorbance when measuring filter member immersion sample/ Absorbance when measuring sample × 100

Table 2 shows the antigen recovery rate when each member was immersed in the sample. For FLU A, FLU B and RS viruses, and adenovirus antigens, the antigen recovery rates were good within 100% ± 15% when any pad member was immersed in the sample. On the other hand, for the SARS-CoV-2 antigen, the antigen recovery rate was significantly reduced when a glass fiber pad was immersed in the sample.

**[Table 1]**

| Antigen | Measurement reagent | Sample amount [µL] | Measurement time [min] |
|---|---|---|---|
| FLU A | RapidTesta FLU/NEXT test device | 120 | 15 |
| FLU B | RapidTesta FLU/NEXT test device | 120 | 15 |
| RS virus | RapidTesta RS V-adeno/NEXT test device | 120 | 10 |
| Adenovirus | RapidTesta RSV-adeno/NEXT test device | 120 | 10 |
| SARS-CoV-2 | Immunochromatography test strip for measuring SARS-CoV-2 | 120 | 15 |

**[Table 2]**

| Antigen | Antigen recovery rate [%] | | | |
|---|---|---|---|---|
| | Glass fiber pad 1 | Glass fiber pad 2 | Polyester fiber pad 1 | Polyester fiber pad 2 |
| FLU A | 89 | 92 | 114 | 100 |
| FLU B | 95 | 106 | 106 | 100 |
| RS virus | 94 | 110 | 101 | 107 |
| Adenovirus | 107 | 111 | 93 | 102 |
| SARS-CoV-2 | 51 | 81 | 102 | 104 |

It was presumed that the contact between the antigen and the glass fiber reduced the amount of antigen that could be subjected to subsequent immunochromatography, resulting in a lower recovery rate.

Table 3 shows the isoelectric points of proteins contained in the antigens used in Example 1 and detected by the measurement reagents, and proteins analogous thereto. The isoelectric points of the proteins were calculated by inputting UniProtKB into Expaxy Compute pI/Mw (https://www.expasy.org/resources/compute-pi-mw). Table 3 reveals that only the isoelectric points of the nucleocapsid proteins of the coronaviruses SARS-CoV-2, MERS-CoV, and SARS-CoV are as high as 9.5 or higher. Glass fiber shows a negative charge when it comes into contact with a neutral sample diluent, whereas the nucleocapsid protein of SARS-CoV-2, which has an isoelectric point of 10.07, has a positive charge. Therefore, the nucleocapsid protein of SARS-CoV-2 is considered to be more easily adsorbed to glass fibers than other antigenic proteins with an isoelectric point of less than 9.5. On the other hand, it is presumed that since polyester does not show an electric charge when it comes into contact with a neutral sample diluent, the antigen protein was not adsorbed to the pad regardless of the isoelectric point of the antigen protein (FIG. 2).

**[Table 3]**

| Antigen | Protein to be detected by reagent | UniProtKB | Isoelectric point |
|---|---|---|---|
| Influenza A virus | Nucleocapsid protein | O91743 (NCAP_I93A0) | 9.35 |
| Influenza B virus | Nucleocapsid protein | P04665 (NCAP_INBLE) | 9.40 |
| RS virus | Fusion glycoprotein F0 | P03420 (FUS_HRSVA) | 9.10 |
| Adenovirus | Hexon protein | P03277 (CAPSH_ADE02) | 5.06 |
| SARS-CoV-2 | Nucleocapsid protein | P0DTC9 (NCAP_SARS2) | 10.07 |
| MERS-CoV | Nucleocapsid protein | K9N4V7 (NCAP_MERS1) | 10.05 |
| SARS-CoV | Nucleocapsid protein | P59595 (NCAP_SARS) | 10.11 |

### <<Production Example 2» Preparation of immunochromatography test strip for FLU/SARS-CoV-2 measurement

### 1) Preparation of anti-FLU monoclonal antibody

Anti-FLU A monoclonal antibodies and anti-FLU B monoclonal antibodies used in the following tests were obtained by immunizing mice with FLU A or FLU B antigens and using methods commonly used by those skilled in the art to produce monoclonal antibodies.

### 2) Preparation of colloidal gold-labeled anti-FLU antibody (conjugate)

Colloidal gold-labeled anti-FLU A antibody solution and colloidal gold-labeled anti-FLU B antibody solution were prepared in the same manner as in <<Production Example 1» "2) Preparation of colloidal gold-labeled anti-SARS-CoV-2 antibody (conjugate)" described above.

### 3) Preparation of conjugate pad

The colloidal gold-labeled SARS-CoV-2 antibody solution, colloidal gold-labeled anti-FLU A antibody solution, and colloidal gold-labeled anti-FLU B antibody solution prepared in 2) were each added at a concentration of 4OD/mL to a solution of 1.33% casein and 4% sucrose (pH 7.5), thereby preparing a conjugate solution. The conjugate solution was applied linearly to a glass fiber pad or a polyester pad, and the conjugate solution was dried to obtain a conjugate pad.

### 4) Preparation of antibody-immobilized membrane

PBS containing 0.75 mg/mL anti-SARS-CoV-2 antibody and 2.5 % sucrose was prepared and used as a SARS-CoV-2 test line coating solution. Similarly, a FLU A test line coating solution and a FLU B test line coating solution were prepared. PBS containing 1.0 mg/mL goat anti-mouse IgG monoclonal antibody and 2.5 % sucrose was prepared and used as a control line coating solution. Using a dispenser for immunochromatography "XYZ3050" (manufactured by BioDot), the test line coating solution and the control line coating solution were each applied on a nitrocellulose membrane at 1.0 µL/cm and dried to obtain an antibody-immobilized membrane. Each line was arranged in the order of FLU A test line, FLU B test line, SARS-CoV-2 test line, and control line from upstream of sample spreading.

### 5) Production of immunochromatography test strip

The antibody-immobilized membrane, the conjugate pad, and an absorbent pad were attached to a plastic adhesive sheet, and the resultant was cut into a 5 mm-width strip to obtain an immunochromatography test strip for measuring FLU/SARS-CoV-2.

### «Example 2» Evaluation 2 of the influence of conjugate pad material on virus antigen measurement sensitivity

Samples used were a sample obtained by adding SARS-CoV-2 Nucleocapsid Recombinant Protein (Icosagen) to the sample diluent attached to RapidTesta FLU/NEXT at a concentration of 450 TCID50/mL, a sample obtained by adding Kitakyusyu 159/93 strain-derived FLU A antigen at a concentration of 6.25 × 10³ TCID50/mL to the sample diluent, and a sample obtained by adding FLU B antigen derived from Lee 40 strain at a concentration of 1.0 × 10⁵ TCID50/mL to the sample diluent. 120 µL of each sample was dropped onto the immunochromatography test strip for measuring FLU/SARS-CoV-2. Ten minutes after dropping the antigen solution, the absorbance of the test line was measured using a RapidTesta Reader (Sekisui Medical Co., Ltd.). The results are shown in Table 4. The absorbance is the average value of N=3 measurements. Absorbance fluctuations in the FLU A and FLU B test lines when changing the conjugate pad material were slight. On the other hand, the SARS-CoV-2 test line absorbance when using the polyester conjugate pad was 1.4 times higher than when using the glass fiber conjugate pad. It is presumed that by using the polyester conjugate pad, the adsorption of the SARS-CoV-2 antigen to the conjugate pad was suppressed, and the amount of antigen reaching the test line increased, resulting in an increase in sensitivity.

**[Table 4]**

| | Absorbance [mAbs] | |
|---|---|---|
| Conjugate pad material | Glass fiber | Polyester |
| FLU A | 32.1 | 37.2 |
| FLU B | 31.0 | 29.9 |
| SARS-CoV-2 | 36.0 | 51.9 |

### «Example 3» Evaluation of the relationship between the position of the conjugate section, the measurement time, and the sensitivity

By varying the position of the conjugate section and the measurement time, sensitivity was evaluated when the contact time between the antigen and the pad was varied.

An immunochromatography test strip for measuring SARS-CoV-2 was prepared according to <<Preparation Example 1>> except that a polyester pad was used in addition to the glass fiber pad as the pad material, and the conjugate was applied linearly at a position 3, 8, or 13 mm from the downstream end of the conjugate pad in the sample spreading direction. The same SARS-CoV-2 sample as in Example 2 was dropped onto the prepared test strip, and the absorbance of the test line was measured after 5, 10, and 15 minutes using a RapidTesta Reader (Sekisui Medical Co., Ltd.). The results are shown in Table 5. The absorbance is the average value of N=3 measurements. With any conjugate position and measurement time, the measurement sensitivity increased by changing the pad material from glass fiber to polyester. The degree of increase in measurement sensitivity by changing the pad material from glass fiber to polyester increased when the conjugate position was far from the downstream end of the conjugate pad, i.e., when the antigen-conjugate complex had a long distance to travel on the conjugate pad. A similar tendency was also observed when the measurement time was short and the amount of antigen passing through the conjugate pad was small.

**[Table 5]**

| | | Sensitivity [mAbs] | | Sensitivity ratio |
|---|---|---|---|---|
| Conjugate line position [mm] | Measurement time [min] | Glass fiber | Polyester | Polyester /glass fiber |
| 13 | 5 | 7.2 | 21.9 | 3.1 |
| 8 | 5 | 10.8 | 21.3 | 2 |
| 3 | 5 | 18.0 | 28.4 | 1.6 |
| 13 | 10 | 20.0 | 52.7 | 2.6 |
| 8 | 10 | 36.0 | 51.9 | 1.4 |
| 3 | 10 | 27.7 | 40.9 | 1.5 |
| 13 | 15 | 39.0 | 69.9 | 1.8 |
| 8 | 15 | 47.8 | 60.4 | 1.3 |
| 3 | 15 | 28.4 | 43.0 | 1.5 |

### «Example 4» Effect of addition of cationic substance on clinical sample measurement sensitivity

Lipidure-BL502 (cationic polymer, NOF Corporation) was dissolved in a sample diluent (containing phosphate buffer) provided with the RapidTesta FLU NEXT so as to give a final concentration of 0.05 %, thereby preparing a sample diluent containing a cationic substance. After suspending each of five positive specimens of SARS-CoV-2 in a sample diluent provided with the RapidTesta FLU/NEXT and the sample diluent containing a cationic substance, the resulting was filtered through a specimen filtration filter provided with the RapidTesta FLU/NEXT to prepare a sample. 120 µL of the sample was dropped onto an immunochromatography test strip for measuring SARS-CoV-2, which was prepared in the same manner as <<Production Example 1» except that a conjugate pad was prepared using a polyester pad, and 10 minutes after dropping the sample, the absorbance of the test line was measured using a RapidTesta Reader (Sekisui Medical Co., Ltd.). The results are shown in Table 6. For all samples, the sensitivity increased when the cationic polymer was added to the sample diluent. Thus, by performing the immunoassay in the presence of a cationic polymer, it was possible to further suppress the adsorption of SARS-CoV-2 nuclear protein having a high isoelectric point to the immunochromatography test strip.

**[Table 6]**

| | Sensitivity [mAbs] | |
|---|---|---|
| | Sample diluent | Cation-added sample diluent |
| Sample 1 | 21.3 | 46.5 |
| Sample 2 | 45.2 | 53.6 |
| Sample 3 | 35.6 | 54.5 |
| Sample 4 | 21.2 | 28.3 |
| Sample 5 | 104.0 | 112.7 |

### [Industrial Applicability]

The present invention can provide an immunochromatography test strip that enables the detection of proteins with an isoelectric point of 9.5 or higher with high sensitivity.

### [Reference Signs List]

11 Glass
11a Silicon atom
11b Oxygen atom
12 Protein with an isoelectric point of 9.5 or higher
13 Polyester or polyolefin
100, 200 Immunochromatography test strip
101, 201 Backing sheet
102, 202, 302 Conjugate pad
103, 203 Absorbent pad
104, 204 Insoluble membrane
105,205 Conjugate section
106, 206 Test line (detector section)
107, 207 Control line
208 Sample pad
305 Linear conjugate section
W Line width

## Claims

1. An immunochromatography test strip for detecting a detection target substance, comprising:
a sample supply section to which a sample potentially containing the detection target substance is supplied;
a conjugate section containing a conjugate in which a label is bound to an antibody or antigen that immunologically reacts with the detection target substance; and
a detector section that captures a complex containing the detection target substance and the conjugate,
wherein the detection target substance is a protein with an isoelectric point of 9.5 or higher, and
either or both of the sample supply section and the conjugate section are formed on a polyester fiber pad or a polyolefin fiber pad.

2. The immunochromatography test strip according to claim 1, wherein either or both of the sample supply section and the conjugate section are formed on a polyester fiber pad.

3. The immunochromatography test strip according to claim 1, which does not include a member made of glass fiber on an upstream side of the detection unit in a sample spreading direction on the immunochromatography test strip.

4. The immunochromatography test strip according to claim 1, wherein the conjugate section is formed within 13 mm from a downstream end of the polyester fiber pad or the polyolefin fiber pad in a sample spreading direction.

5. The immunochromatography test strip according to claim 1, wherein the conjugate section is linearly formed on the polyester fiber pad.

6. The immunochromatography test strip according to claim 1, wherein the protein with an isoelectric point of 9.5 or higher is derived from a respiratory infection virus.

7. The immunochromatography test strip according to claim 6, wherein the respiratory infection virus is a coronavirus.

8. The immunochromatography test strip according to claim 7, wherein the coronavirus is SARS-CoV-2.

9. The immunochromatography test strip according to claim 1, further comprising a cationic substance.

10. The immunochromatography test strip according to claim 9, wherein the cationic substance is at least one selected from a cationic polymer, a cationic surfactant, and a metal salt.

11. An immunochromatography kit comprising a specimen diluent containing a cationic substance and the immunochromatography test strip of claim 1.

12. The immunochromatography kit according to claim 11, wherein the cationic substance is at least one selected from a cationic polymer, a cationic surfactant, and a metal salt.

13. An immunochromatography kit comprising a sample filtration filter including a member formed of polyester fiber or polyolefin fiber, and the immunochromatography test strip of claim 1.

14. An immunoassay method for a protein having an isoelectric point of 9.5 or higher, comprising using the immunochromatography test strip of claim 1 or the immunochromatography kit of claim 11.

15. A filtration method for a sample containing a detection target substance, the method comprising:
a step of filtering the sample with a filter member for sample filtration including a filter formed of polyester fiber or polyolefin fiber,
wherein the detection target substance is a substance with an isoelectric point of 9.5 or higher.

16. An immunoassay method for a detection target substance, comprising a step of measuring the substance with an isoelectric point of 9.5 or higher in a sample prepared by the filtration method of claim 15 by immunochromatography,
wherein the substance with an isoelectric point of 9.5 or higher is a protein with an isoelectric point of 9.5 or higher.
